**Europäisches Patentamt**

(19) **European Patent Office**

**Office européen des brevets**

(11) Publication number : **0 408 145 B1**

## (12) EUROPEAN PATENT SPECIFICATION

(45) Date of publication of patent specification :
**12.01.94 Bulletin 94/02**

(51) Int. Cl.⁵ : **B01L 3/14, B01L 3/00**

(21) Application number : **90201861.3**

(22) Date of filing : **10.07.90**

(54) **Containers.**

(30) Priority : **14.07.89 US 380843**

(43) Date of publication of application :
**16.01.91 Bulletin 91/03**

(45) Publication of the grant of the patent :
**12.01.94 Bulletin 94/02**

(84) Designated Contracting States :
**BE CH DE DK FR GB IT LI LU NL SE**

(56) References cited :
**EP-A- 0 347 837**
**EP-A- 0 408 144**
**WO-A-82/00024**
**WO-A-86/03011**

(56) References cited :
**US-A- 3 419 179**
**US-A- 3 854 883**
**US-A- 4 473 530**
**US-A- 4 639 419**

(73) Proprietor : **EASTMAN KODAK COMPANY**
**343 State Street**
**Rochester, New York 14650-2201 (US)**

(72) Inventor : **Zander, Dennis R., c/o EASTMAN**
**KODAK COMPANY**
**Patent Department, 343 State Street**
**Rochester, New York 14650 (US)**

(74) Representative : **Phillips, Margaret Dawn et al**
**Kodak Limited Patent Department Headstone**
**Drive**
**Harrow, Middlesex HA1 4TY (GB)**

## Description

This invention relates to containers and in particular containers having reagents deposited in a closed end and means for preventing the dislodging of the reagents.

Examples of the state of the art of placing dried reagents in the closed end of a container are found in US Patent Specification USA-4639419 entitled "Immunological Color Change Test Involving Two differently Colored Reagent Spots" issued on January 27, 1987 and US Patent Specification US-A-4673639 entitled "Dry Form Micronitrous Acid Streptococci Extraction-Agglutination Test" issued on June 16, 1987. To date, these containers have been less than completely satisfactory since their construction has ignored the fact that if the closed end strikes an object, the reagent can be dislodged. Furthermore, even if the dislodging problem is acknowledged, it is not sufficient as the solution to the problem, to simply construct the bottom portions of the container to be much thicker, since at least the side wall portion adjacent the bottom wall of the container must be sufficiently flexible as to allow the container to be squeezed right above the bottom wall. Such squeezing is used to redissolve the stored reagents more rapidly.

It is therefore an object of the present invention to provide an improved container with a means for preventing dislodging of the reagent when the closed end strikes an object, without affecting the flexibility of the container.

More specifically, in accordance with one aspect of the present invention, there is provided a container having a sidewall, a bottom wall joined to the sidewall at a junction point, and a reagent deposited on an inner surface of the bottom wall;

characterized in that the container further includes absorber means extending from the bottom wall at or below the junction point for absorbing shock, the container and the absorber means comprising a material which is sufficiently flexible as to allow the sidewall adjacent the bottom wall to be squeezed by finger pressure.

It is an advantageous feature of the invention that a container is provided which includes an absorber means preventing subsequent dislodging of deposited reagents located in the closed end.

It is a related advantageous feature of the invention that such absorber means are provided without significantly interfering with the squeezability of the container.

The present invention will now be described by way of example only with reference to the accompanying drawings in which:-

Figure 1 is a sectioned elevation view of a container constructed in accordance with the present invention;

Figure 2 is a fragmentary sectioned view taken generally along the line II-II of Figure 1;

Figure 3 is a sectioned elevation view similar to that illustrated in Figure 1 but illustrating a second embodiment of a container contructed in accordance with the present invention; and

Figures 4 to 6 are fragmentary sectioned views similar to that illustrated in Figures 1 or 3, but illustrating further embodiments of containers contructed in accordance with the present invention.

The invention is described hereinafter in connection with certain preferred embodiments wherein the container is particularly suited for absorption of shock at its closed end through use of reinforcing means extending from the closed end. Thus, plastics materials are preferred for the construction of such a container.

The terms "above", "below" and the like refer to orientations of parts when the device is in its preferred orientation for use, that is, when the container is generally vertical with its open end up.

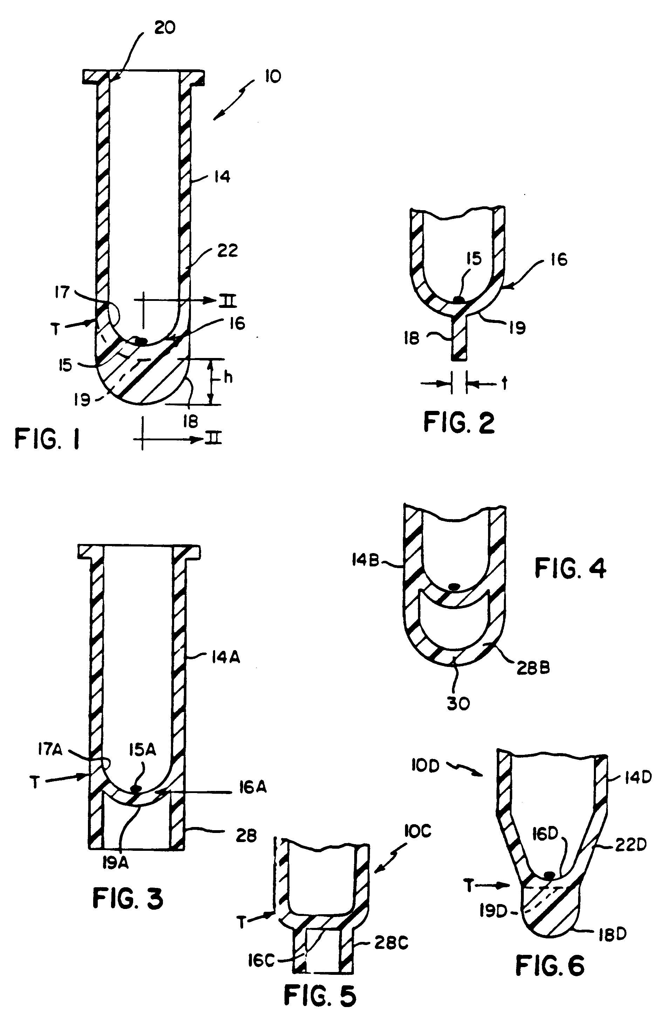

As discussed hereinafter, the preferred embodiment illustrated in Figure 1 is a container 10 having a side wall 14 which is preferably cylindrical, a closed end provided by a bottom wall 16 joined to side wall 14 at junction T and at least one reagent 15 deposited on inner surface 17 of bottom wall 16. Preferably, opposite end 20 is an open end of conventional construction which can be stoppered. At least portions 22 of side wall 14 are flexible, namely, the portion adjacent to junction T in order to allow container 10 to be squeezed. This encourages the redissolving of reagent 15 when water or sample is added to the container 10. Reagent(s) 15 can be present for any purpose including extraction of chlamydial, gonococcal or herpes antigen as disclosed in commonly owned European patent application EP-A-408144 filed on even date herewith and entitled "Extracting Device for Extracting Antigens", wherein the preferred reagents for depositing and drying in the extraction container are dithiothreitol, a reducing agent, with polyacralamide, a stabilizer and TRISMA (tris-hydroxyaminomethane).

In US patent application no. 255,928 filed on October 7, 1988 and entitled "High pH Extraction Composition And Its Use To Determine A Chlamydial Gonococcal or Herpes Antigen", a composition is disclosed which is useful for extracting antigen from chlamydial, gonococcal or herpes organisms having a pH of at least about 8 and comprising a strong base and an alcoholamine. Other addenda preferably included in the extraction composition include a cationic surfactant, one or more reducing agents, preservatives to prevent hydrogen peroxide activity and chelating agents. It is conceivable that either the strong base, the alcoholamine, the cationic surfactant or the reducing agent could be used for the same purpose.

Examples of other reagents which could be used for other purposes in this invention are enzymes, enzyme substrates, antibodies, antigens, haptens, inorganic and organic reagents, buffers, salts and the like, as well as radioactively tagged or fluorescent reagents of the foregoing types including nonisotopic tags such as enzymes, cofactors, luminescent agents and the like as disclosed in US Patent Specification US-A-4234316 entitled "Device for Delivering Measured Quantities of Reagents into Assay Medium" issued on November 18, 1980.

In further accordance with the invention, the container is improved by absorber means 18 (Figure 1) which extend from bottom wall 16 at or below junction T. This location of the absorber means 18 is considered critical, since locating it above the junction tends to interfere with the flexibility of portions 22 of side wall 14. The reinforcing means 18 preferably comprise a flexible flange extending from surface 19 of wall means 14 approximately at the middle of bottom wall 16. The flange can be constructed by molding flexible material consistent or compatible with that used in the construction of the wall means 14 onto the closed end. The height h (Figure 1) and the thickness t (Figure 2) of flange 18 need to be controlled as described hereinafter, to provide effective shock resistance.

Although flange 18 is shown curved, it can also have right-angle corners, not shown.

Alternate embodiments are illustrated in Figures 3 to 5. Parts similar to those previously described bear the same reference numeral to which the distinguishing suffix "A", "B" or "C" is appended.

Thus, the container illustrated in Figure 3 similarly comprises side wall 14A joined to bottom wall 16A at junction T and at least one reagent 15A deposited inside of bottom wall 16A. However, the absorbing means which extends from bottom wall 16A, is a flexible skirt 28 extending downward from surface 19A, opposite to inner surface 17A. This skirt can be constructed with a variety of shapes. As shown, it preferably extends around the circumference of closed end 16A.

In Figure 4, the skirt joins itself to form a shock-absorbing bubble 28B. This can be fabricated by joining skirt portions together at junction 30.

In Figure 5, skirt 28C is joined to wall 16C below junction T.

Variations of these embodiments could include one or more thin, flexible prongs (not shown) extending from the closed end of the tube.

The sidewall of the container can have a compound shape as illustrated in Figure 6. Parts similar to those previously described bear the same reference numeral, to which the distinguishing suffix "D" has been appended.

Thus, container 10D has a sidewall 14D joined to a bottom wall 16D at junction T, and an absorber flange 18D projecting from outside surface 19D. However, sidewall 14D has a portion 22D, which preferably is the squeezable flexible portion, which is conically shaped rather than cylindrically shaped like the rest of side wall 14D.

To demonstrate the effect that the height and thickness of the absorber means have, on its function, the embodiment of Figures 1 and 2 was tested as given in the following examples. (Similar constraints apply to the skirt or bubble configurations shown, albeit different values may apply.)

Example 1

In this example, a set of 100 identical polyethylene tubes, 7.57cm long from the top of the open end 20 to the bottom of the absorber means 18, as shown in Figure 1, was dropped at a height of 68.6cm from a solid flooring. (The 68.6cm is measured from the bottom of the absorber means, and is equivalent to 30 inches measured from the top open end.) The flange 18 was 2.54mm (0.1 inch) long and 1.0mm thick, and the sidewall 14 had a thickness of about 0.5mm. Each tube had a dried reagent deposit as shown in Figure 1, which weighed about 0.016g, with a total tube weight of about 1.716g. The set of tubes was dropped five separate times, and the cumulative number of times a tube failed was noted, that is, the dried reagent was dislodged. The results are shown in Table I below. No tube failed until the fifth run-through, when one tube failed. A set of tubes is considered a success if none of a set of 100 tubes failed on the first drop, and no more than two out of 100 failed on the second drop.

## Table I

| # of Times Each Tube Dropped | Cumulative # of Failures out of 100 |
|---|---|
| 1 | 0 |
| 2 | 0 |
| 3 | 0 |
| 4 | 0 |
| 5 | 1 |

### Example 2

A set of 50 tubes was prepared and dropped as in Example 1, except that flange 18 was reduced in length so as to be only about 1.5mm long. The results obtained are shown in Table IIA.

## Table IIA

| # of Times Each Tube Dropped | Cumulative # of Failures out of 50 | # of Failures for 100* |
|---|---|---|
| 1 | 0 | 0 |
| 2 | 0 | 0 |
| 3 | 0 | 0 |
| 4 | 1 | 2 |
| 5 | 4 | 8 |

\* Extrapolated from the column for 50.

For comparison purposes, a set of comparative examples was prepared by repeating Example 2, except that the flange 18 was cut off to leave only a suggestion of its presence - a length of less than about 0.5mm. The results obtained are shown in Table IIB, and represent a failed set.

## Table IIB — Comparative Example

| # of Times Each Tube Dropped | Cumulative # of Failures out of 50 | # of Failures for 100* |
|---|---|---|
| 1 | 0 | 0 |
| 2 | 6 | 12 |
| 3 | 9 | 18 |
| 4 | 10 | 20 |
| 5 | 10 | 20 |

\* Extrapolated from the column for 50.

### Example 3

A set of 50 tubes was prepared and dropped as in Example 1, except that flange 18 was reduced in thickness to a value of only about 0.5mm. The results obtained are shown in Table III.

## Table III

| # of Times Each Tube Dropped | Cumulative # of Failures out of 50 | # of Failures for 100* |
|---|---|---|
| 1 | 0 | 0 |
| 2 | 1 | 2 |
| 3 | 2 | 4 |
| 4 | 6 | 12 |
| 5 | 9 | 18 |

\* Extrapolated from column for 50.

Thus the flange 18 can be as thin as about 0.5mm, and still provide adequate shock absorbance under this test.

### Example 4 - Repeat of Example 1

A set of 50 tubes was prepared and dropped identical to Example 1, but by a different experimenter, to see if the experiment was sensitive to the person doing the testing. The results obtained are shown in Table IV:

## Table IV

| # of Times Each Tube Dropped | Cumulative # of Failures out of 50 | # of Failures for 100* |
|---|---|---|
| 1 | 0** | 0 |
| 2 | 1** | 2 |
| 3 | 3** | 6 |
| 4 | 8** | 16 |
| 5 | 13** | 26 |

\* Extrapolated from column for 50.

\*\* Substantially higher failure rates occur if the distance dropped is significantly larger than 68.2 cm, e.g., if it is at least 81.3 cm.

The results of Table IV are considered to be within experimental error of the results of Table I.

## Claims

1. A container (10; 10C; 10D) having a sidewall (14; 14A; 14B; 14D), a bottom wall (16; 16A; 16C; 16D) joined to the sidewall (14; 14A; 14B; 14D) at a junction point (T), and a reagent (15) deposited on an inner surface (17) of the bottom wall (16; 16A; 16C; 16D);
   characterized in that the container (10; 10C; 10D) further includes absorber means (18; 28; 28B; 28C; 18D) extending from the bottom wall (16; 16A; 16C; 16D) at or below the junction point (T) for absorbing shock and preventing the shock from being transmitted to the bottom wall, the container (10; 10C; 10D) and the absorber means (18; 28; 28B; 28C; 18D) comprising a material which is sufficiently flexible as to allow the sidewall (14; 14A; 14B; 14D) adjacent the bottom wall (16; 16A; 16C; 16D) to be squeezed by finger pressure.

2. A container according to claim 1, wherein the sidewall (14; 14A; 14B; 14D), the absorber means (18; 28; 28B; 28C; 18D) and the bottom wall (16; 16A; 16C; 16D) are formed from polyethylene.

3. A container according to claim 1 or 2, wherein the absorber means comprises a flexible flange (18; 18D) extending from the bottom wall (16; 16D) approximately at its middle.

4. A container according to claim 1 or 2, wherein the absorber means comprise a flexible skirt (28; 28C) extending from a surface of the bottom wall (16A; 16C).

5. A container according to claim 4, wherein the skirt (28; 28C) extends around the entire circumference of the bottom wall (16A; 16C).

6. A container according to claim 4 or 5, wherein the skirt portions (28C) join together approximately at the middle (30) of the bottom wall forming a shock-absorbing bubble.

7. A container according to any one of claims 1 to 3, wherein the absorber means (18; 28; 28B; 28C; 18D) has a length and thickness sufficient to prevent a dried reagent weighing 0.016 g deposited onto the inner surface (17) from being dislodged when the container (10) is dropped bottom wall first a distance of 68.6cm onto a hard surface with a container weight of 1.716 g.


**Patentansprüche**

1. Behälter (10; 10C; 10D) mit einer Seitenwandung (14; 14A; 14B; 14D), einer an einem Anschlußpunkt (T) mit der Seitenwandung (14; 14A; 14B; 14D) verbundenen Bodenwandung (16; 16A; 16C; 16D) und einem auf eine Innenfläche (17) der Bodenwandung (16; 16A, 16C; 16D) aufgebrachten Reagenz (15), **dadurch gekennzeichnet, daß**
der Behälter (10; 10C; 10D) Stoßdämpfungsmittel (18; 28; 28B; 28C; 18D) enthält, die sich von dem Anschlußpunkt (T) aus oder unterhalb dieses Punktes (T) von der Bodenwandung (16; 16A; 16C; 16D) weg erstrecken und verhindern, daß Erschütterungen auf die Bodenwandung übertragen werden, und daß der Behälter (10; 10C; 10D) und die Stoßdämpfungsmittel (18; 28; 28B; 28C; 18D) aus einem Material bestehen, das so flexibel ist, daß sich die Seitenwandung (14; 14A; 14B; 14D) benachbart der Bodenwandung (16; 16A; 16C; 16D) durch Fingerdruck zusammenpressen läßt.

2. Behälter nach Anspruch 1, dadurch gekennzeichnet, daß die Seitenwandung (14; 14A; 14B; 14D), die Stoßdämpfungsmittel (18; 28; 28B; 28C; 18D) und die Bodenwandung (16; 16A, 16C; 16D) aus Polyäthylen hergestellt sind.

3. Behälter nach Anspruch 1 oder 2, dadurch gekennzeichnet, daß die Stoßdämpfungsmittel einen flexiblen Ansatz (18; 18D) umfassen, der ungefähr aus der Mitte der Bodenwandung (16; 16D) ragt.

4. Behälter nach Anspruch 1 oder 2, dadurch gekennzeichnet, daß die Stoßdämpfungsmittel einen flexiblen Sockel (28; 28C) umfassen, der aus einer Fläche der Bodenwandung (16A; 16C) ragt.

5. Behälter nach Anspruch 4, dadurch gekennzeichnet, daß der Sokkel (28; 28C) um den gesamten Umfang der Bodenwandung (16A; 16C) herum verläuft.

6. Behälter nach Anspruch 4 oder 5, dadurch gekennzeichnet, daß die Sockelteile (28B) ungefähr in der Mitte (30) der Bodenwandung miteinander verbunden sind und eine Erschütterungen absorbierende Blase bilden.

7. Behälter nach einem der Ansprüche 1 bis 3, dadurch gekennzeichnet, daß die Stoßdämpfungsmittel (18; 28; 28B; 28C; 18D) so lang und dick sind, daß ein auf der Innenfläche (17) lagerndes Trockenreagenz mit einem Gewicht von 0,016 g nicht aus seiner Lage verschiebbar ist, wenn der Behälter (10), der ein Gewicht von 1,716 g besitzt, mit der Bodenwandung voran aus einer Höhe von 68,6 cm auf eine harte Fläche fallen gelassen wird.

**Revendications**

1. Récipient (10 ; 10C ; 10D) comportant une paroi latérale (14 ; 14A ; 14B ; 14D), une paroi inférieure (16 ; 16A ; 16C ; 16D) raccordée à la paroi latérale (14 ; 14A ; 14B ; 14D) au niveau d'un point de raccord (T), et un réactif (15) déposé sur une surface interne (17) de la paroi inférieure (16 ; 16A ; 16C ; 16D) ; caractérisé en ce que le récipient (10 ; 10C ; 10D) comporte de plus des moyens absorbeurs (18 ; 28 ; 28B ; 28C ; 18D) se prolongeant à partir de la paroi inférieure (16 ; 16A ; 16C ; 16D) au niveau du point de raccord ou au-dessous du point de raccord (T) pour absorber les chocs et empêcher les chocs d'être transmis à la paroi inférieure, le récipient (10 ; 10C ; 10D) et le moyen absorbeur (18 ; 28 ; 28B ; 28C ; 28D) comprenant un matériau qui est suffisamment flexible de façon à permettre à la paroi latérale (14 ; 14A ; 14B ; 14D) contiguë à la paroi inférieure (16 ; 16A ; 16C ; 16D) d'être serrée par la pression des doigts.

2. Récipient selon la revendication 1, dans lequel la paroi latérale (14 ; 14A ; 14B ; 14D), le moyen absorbeur (18 ; 28 ; 28B ; 28C ; 18D) et la paroi inférieure (16 ; 16A ; 16C ; 16D) sont formés à partir du polyéthylène.

3. Récipient selon la revendication 1 ou 2, dans lequel le moyen absorbeur comprend un rebord flexible (18 ; 18D) se prolongeant à partir de la paroi inférieure (16 ; 16D) approximativement en son milieu.

4. Récipient selon la revendication 1 ou 2, dans lequel les moyens absorbeurs comprennent une bordure flexible (28 ; 28C) se prolongeant à partir d'une surface de la paroi inférieure (16A ; 16C).

5. Récipient selon la revendication 4, dans lequel la bordure (28 ; 28C) s'étend autour de la circonférence entière de la paroi inférieure (16A ; 16C).

6. Récipient selon la revendication 4 ou 5, dans lequel les parties de bordure (28C) se raccordent ensemble approximativement au milieu (30) de la paroi inférieure formant une bulle absorbant les chocs.

7. Récipient selon l'une quelconque des revendications 1 à 3, dans lequel le moyen absorbeur (18 ; 28 ; 28B ; 28C ; 18D) présente une longueur et une épaisseur suffisantes pour empêcher un réactif sec pesant 0,016 g déposé sur la surface interne (17) d'être détaché lorsque le récipient (10) tombe, sa paroi inférieure la première d'une hauteur de 68,6 cm sur une surface dure, le poids du récipient étant de 1, 716 g.

FIG. 1

FIG. 2

FIG. 3

FIG. 4

FIG. 5

FIG. 6

8